# NOUVEAU FASCICULE DE BREVET EUROPEEN

(11) **EP 2 257 476 B2**
(45) Date de publication et mention de la décision concernant l'opposition: **24.02.2021**
(45) Mention de la délivrance du brevet: 14.03.2012
(21) Numéro de dépôt: 09734605.0
(22) Date de dépôt: 27.03.2009
(51) Int. Cl.: B65D 47/18, B65D 47/20, A61F 9/00, A61J 1/14, B05B 11/04, B05B 11/00

(54) **DISPOSITIF DE DISTRIBUTION DE LIQUIDE SOUS FORME DE GOUTTES**
VORRICHTUNG ZUR AUSGABE EINER FLÜSSIGKEIT IN TROPFENFORM
DEVICE FOR DISPENSING A LIQUID IN THE FORM OF DROPS

(30) Priorité: 27.03.2008 FR 0851994; 15.10.2008 FR 0805717
(43) Date de publication de la demande: 08.12.2010
(73) Titulaire: NEMERA LA VERPILLIERE, 38290 La Verpillière (FR)
(72) Inventeur: PAINCHAUD, Gaétan, F-69340 Francheville (FR); LANZI, Sylvain, F-38850 Chirens (FR); JULIA, Xavier, F-38090 Villefontaine (FR); GREVIN, Guillaume, F-38080 L'Isle d'Abbeau (FR)
(74) Mandataire: Vallée-Thiollier, Clémence-Olivia Laure Marie
(86) Numéro de dépôt international: PCT/FR2009/000356
(87) Numéro de publication internationale: WO 2009/130411

(56) Documents cités:
- EP-A- 1 561 699
- EP-A2- 0 985 454
- EP-A2- 1 787 605
- EP-B1- 1 453 738
- WO-A-2006/043295
- WO-A1-2004/067400
- WO-A1-2009/130412
- DE-A1-102006 012 898
- FR-A1- 2 929 249
- JP-A- 7 223 662
- JP-A- 2004 001 829
- US-A- 5 431 310
- US-A1- 2002 017 294
- US-A1- 2006 261 098

## Description

La présente invention concerne le domaine technique de la distribution de doses prédéterminées. Plus précisément mais non exclusivement, elle concerne un dispositif de distribution selon le préambule de la revendication 1. Un tel document est connu du document EP 1 561 699 A.

On connaît déjà dans l'état de la technique des dispositifs permettant de mettre en œuvre cette distribution sous forme de gouttes. Généralement, afin de former les gouttes délivrées, la partie supérieure de la tête de distribution du dispositif comporte une forme dont le volume permet de définir la goutte.

La présente invention vise notamment à proposer un dispositif distribuant des doses prédéterminées de liquide tout en assurant une meilleure stérilité du liquide distribué.

A cet effet, l'invention a pour objet un dispositif de distribution de doses prédéterminées de liquide, comportant un organe d'étanchéité pouvant prendre une position de libération de liquide, laissant sortir du liquide hors du dispositif, et une position anti-retour empêchant le retour de liquide à l'intérieur du dispositif, dans lequel l'organe d'étanchéité comporte des moyens de dosage du liquide à distribuer, les moyens de dosage étant des moyens de formation de gouttes de liquide, le dispositif comportant un canal de passage de liquide débouchant sur les moyens de formation de gouttes, **caractérisé en ce que** les moyens de formation de gouttes comprennent une forme évasée depuis ce canal, le dispositif comprenant un embout destiné à être rapporté sur un réservoir en matière plastique contenant le liquide à distribuer, l'embout comprenant l'organe d'étanchéité, une première partie et une deuxième partie, l'organe d'étanchéité étant disposé entre la première partie et la deuxième partie.

Cette forme évasée permet d'éviter les jets de liquide.

Généralement, les doses prédéterminées de liquide sont des gouttes de liquide. On notera par ailleurs que la position anti-retour est équivalente à une position de blocage du liquide.

Grâce à cette disposition, on ménage les moyens de dosage du liquide directement sur l'organe d'étanchéité, si bien que le liquide libéré par l'organe d'étanchéité en position de libération, sortant tout juste de la zone "étanche" du dispositif, est directement reçu dans les moyens de dosage, sans passer dans d'autres pièces. On diminue ainsi les risques que le liquide s'infiltre dans d'autres parties du dispositif après sa libération par l'organe d'étanchéité. En effet, dans le cas où les moyens de dosage sont prévus sur une autre pièce que sur l'organe d'étanchéité, par exemple sur l'enveloppe extérieure de l'embout de distribution du dispositif, le liquide peut s'infiltrer dans d'autres pièces du dispositif lors de sa circulation entre l'organe d'étanchéité et les moyens de dosage. Or, une telle infiltration de liquide dans des zones non désirées est source de contamination, notamment par développement de bactéries. Ou alors, pour éviter ce risque, il est nécessaire de prévoir des moyens spécifiques pour assurer l'étanchéité.

Par ailleurs, l'intégration des moyens de dosage dans l'organe d'étanchéité rend plus facile la diminution de "volume mort" susceptible de contenir du liquide contaminé. Ce volume mort correspond au volume situé entre la zone étanche (délimitée notamment par le réservoir et l'organe d'étanchéité) et les moyens de dosage de la goutte. Comme ces moyens de dosage sont sur l'organe d'étanchéité, ils sont plus proches de la zone étanche, ce qui restreint le volume mort. En outre, on peut réaliser l'organe d'étanchéité de façon qu'il "colle" le plus possible aux pièces afin de diminuer encore le volume mort. De plus, comme l'organe d'étanchéité est généralement partiellement ou intégralement réalisé en matériau souple, il est plus aisé de diminuer les espaces pour qu'il colle aux pièces que lorsque le volume mort est délimité par des pièces rigides.

L'invention peut en outre comporter l'une ou plusieurs des caractéristiques suivantes.
- Le dispositif comporte des moyens d'appui sur l'organe d'étanchéité, au droit d'une zone évidée, assurant la déformation par flexion de l'organe d'étanchéité pour qu'il prenne sa position anti-retour.
- L'organe d'étanchéité est au moins partiellement souple, et le dispositif comporte des moyens d'appui sur l'organe d'étanchéité, assurant la déformation par compression de la partie souple de l'organe d'étanchéité pour qu'il prenne sa position anti-retour. La partie souple peut comprendre un matériau élastomère ou un matériau suffisamment souple pour assurer l'étanchéité.
- La forme évasée débouche sur une forme sensiblement cylindrique. Cette forme cylindrique permet de calibrer les gouttes formées.
- L'organe d'étanchéité est un élément élastomère, intégralement réalisé en matériau élastomère. Néanmoins, l'organe peut également être réalisé différemment, notamment dans un matériau plastique suffisamment souple.
- L'organe d'étanchéité comprend une partie élastomère et une partie rigide, ces parties étant solidaires l'une de l'autre en déplacement. La présence de la partie rigide permet à cette dernière de recevoir d'éventuelles contraintes exercées sur l'organe d'étanchéité, en évitant de déformer la partie élastomère, dont l'étanchéité est donc assurée de façon plus constante dans le temps.
- Les moyens de dosage sont réalisés dans la partie rigide.
- La partie de l'organe d'étanchéité comportant les moyens de dosage est disposée au voisinage immédiat d'un orifice de libération du liquide hors du dispositif.

L'invention sera mieux comprise à la lecture de la description qui va suivre, donnée uniquement à titre d'exemple et faite en se référant aux dessins dans lesquels :
- la figure 1 est une vue schématique illustrant fonctionnellement l'organe d'étanchéité d'un dispositif selon un mode de réalisation de l'invention ;
- la figure 2 est une vue en coupe longitudinale illustrant un exemple de dispositif tel que schématisé sur la figure 1 ; et
- la figure 3 est une vue analogue à la figure 2, illustrant une variante de l'exemple de la figure 2.

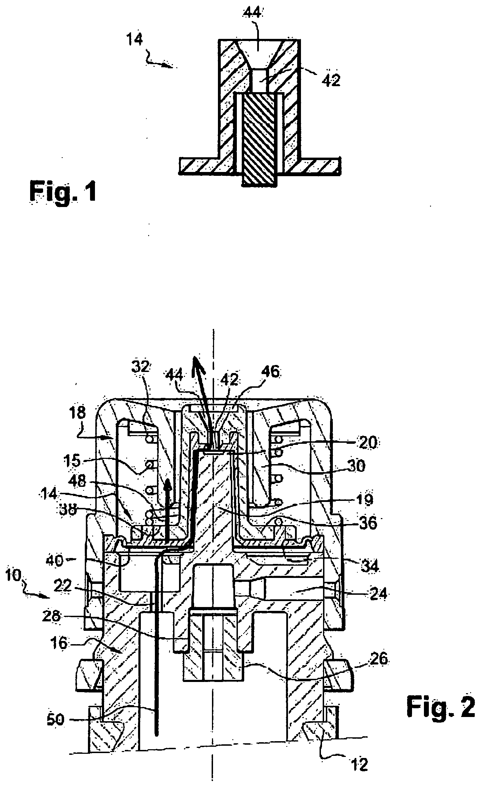

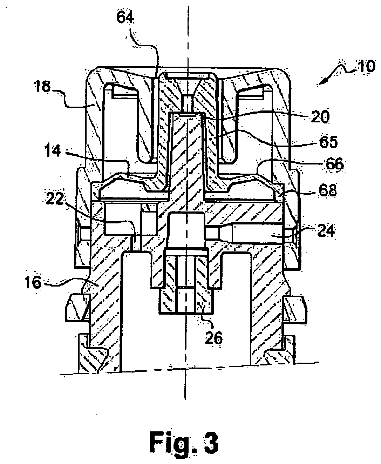

Un dispositif de distribution de liquide comprend un embout 10, dont un exemple est représenté sur les figures 2 et 3, destiné à être rapporté sur un réservoir en matière plastique contenant le liquide à distribuer. Le dispositif permet de distribuer des doses prédéterminées de liquide, plus précisément des gouttes de liquide, destinées à une application oculaire, nasale ou buccale, par exemple des gouttes de collyre pour les yeux. L'embout 10 est rapporté sur un réservoir 12, plus précisément sur le col 12 du réservoir, ce réservoir étant destiné à être pressé par l'utilisateur pour faire sortir le liquide. Dans cet exemple, le réservoir 12 est en matière plastique et est destiné à être pressé par l'utilisateur pour faire sortir le liquide. On peut envisager d'autres types de réservoir, notamment en verre ou en métal, l'utilisateur pouvant libérer du liquide par une autre action qu'en le pressant, par exemple en appuyant sur un élément d'activation d'une pompe.

L'embout 10 comprend un organe d'étanchéité 14, disposé entre une première partie 16 et une deuxième partie 18 de l'embout 10. Cet organe d'étanchéité 14, ou valve d'étanchéité, peut prendre une position de blocage du liquide, ou position anti-retour, illustrée sur les figures 2 et 3, empêchant le retour du liquide une fois sorti de la zone étanche, et une position de libération du liquide (non représentée). De préférence, l'organe 14 est disposé au voisinage de l'extrémité distale de l'embout 10, au voisinage d'un orifice 64 de libération de liquide (voir figure 3). Comme on peut le voir sur le schéma de la figure 1, l'organe d'étanchéité 14 comporte des moyens de dosage du liquide à distribuer 44 décrits dans la suite.

L'embout 10 est recouvert d'un capuchon de fermeture, non représenté sur les figures.

La configuration de l'embout 10 selon un mode de réalisation particulier va être décrit plus précisément, en références aux figures 2 et 3. L'exemple de la figure 3 correspond à une légère variante de celui de la figure 2. En effet, sur la figure 2, l'organe d'étanchéité 14 est en deux parties 34, 36 et est maintenu en position de blocage à l'aide d'un élément de rappel 15, alors que sur la figure 3, l'organe 14 est intégralement réalisé en matériau élastomère et est maintenu en position de blocage par déformation, comme cela est décrit dans la suite.

Comme cela est représenté sur la figure 2, la première partie 16 de l'embout 10 est un noyau intérieur 16, comprenant une protubérance 19 de forme sensiblement cylindrique et faisant saillie de l'extrémité distale du noyau 16. Sur son extrémité distale, cette protubérance 19 porte des moyens 20 d'appui sur l'organe d'étanchéité 14, ces moyens 20 étant dans cet exemple composés d'une saillie formant un bourrelet annulaire destinée à appuyer sur l'organe d'étanchéité 14. De façon alternative, les moyens d'appui 20 pourraient être composés de l'extrémité seule de la protubérance 19, sans inclure une saillie sur cette extrémité. Le noyau intérieur 16 comporte par ailleurs un canal 22 de passage du liquide depuis le réservoir vers l'extérieur du dispositif, ainsi qu'une partie de connexion du noyau 16 au réservoir 12. L'embout 10 comporte par ailleurs des moyens d'évent, destinés à faire passer de l'air à l'intérieur du réservoir pour combler une dépression générée par la sortie du liquide. Dans cet exemple, les moyens d'évent sont portés par le noyau intérieur 16 et comprennent un canal 24 de passage de l'air, traversé par un filtre hydrophobe 26, destiné à filtrer l'air entrant sans pour autant permettre à du liquide de s'échapper par le canal 24. Plus précisément dans cet exemple, le filtre 26 est disposé dans un logement 28 de réception du filtre, ce logement 28 prenant la forme d'une rainure annulaire disposée au centre de la surface intérieure du noyau 16, dans laquelle le filtre 26 est encastré.

La deuxième partie 18 de l'embout 10 correspond, dans cet exemple, à une enveloppe supérieure extérieure de l'embout 10. Cette enveloppe extérieure 18 est destinée à coiffer le noyau intérieur 16, l'organe d'étanchéité 14 (au moins partiellement) et le ressort 15. Plus précisément, elle comprend une protubérance intérieure ouverte 30, formée par une rainure annulaire centrale débouchant sur l'orifice 64, destinée à entourer l'extrémité distale de la protubérance 18 et de l'organe d'étanchéité 14 de façon à permettre le passage de liquide hors du dispositif. L'enveloppe 18 comporte en outre un siège 32 d'appui de l'élément de rappel 15, ce siège 32 étant disposé autour de la protubérance 30. Dans cet exemple, l'extrémité distale de l'organe 14, extrémité par laquelle le liquide est délivré, dépasse légèrement de l'orifice 64. On pourrait envisager que cette extrémité arrive à affleurement de la surface de l'orifice 64, ou soit en retrait à l'intérieur de l'embout 10. Ou encore, on pourrait envisager que l'extrémité de l'organe 14 dépasse davantage de la surface de l'enveloppe 18, ce qui permet d'isoler plus facilement les gouttes par rapport à la surface de l'enveloppe 18.

L'organe d'étanchéité comporte dans cet exemple une partie élastomère 34 et une partie rigide 36, les parties 34 et 36 étant solidaires l'une de l'autre en déplacement, c'est-à-dire que lorsque la partie 34 se déplace, la partie 36 se déplace également avec elle, et vice-versa. Dans cet exemple, les parties 34 et 36 sont assemblées par surmoulage, mais l'on pourrait envisager d'autres types d'assemblage. La partie élastomère 34 est réalisée dans un matériau élastomère, tel que du silicone ou un matériau thermoplastique élastomère. La partie rigide 36 est réalisée dans un matériau plastique tel que du polypropylène. La partie rigide 36 comporte une surface 38 d'appui de l'élément de rappel 15. Comme on peut le constater sur les figures, la partie rigide 36 recouvre la partie élastomère 34 sur sensiblement toute sa surface, une zone 40 de la partie élastomère étant néanmoins laissée libre à l'extrémité de la partie élastomère, de façon à permettre l'allongement de cette partie élastomère 34. Plus précisément, la partie élastomère 34, ainsi que la partie rigide 36, ont chacune la forme d'un chapeau muni d'une forme centrale cylindrique, de forme sensiblement complémentaire à celle de la protubérance 19 du noyau 16, cette forme cylindrique étant prolongée sur son extrémité proximale par un rebord. Ainsi, la partie rigide 36 recouvre la partie élastomère 34 sur une grande partie de sa surface, sauf au niveau de sa périphérie 40. Comme on peut le voir sur les figures, les parties 34, 36 définissent un canal 42, ménagé sur le fond de leur forme centrale cylindrique, permettant au liquide de sortir. Par ailleurs, l'organe d'étanchéité 14 comporte des moyens 44, 46 de dosage du liquide à distribuer, ces moyens étant des moyens de formation de gouttes de liquide. Plus précisément, ces moyens sont formés dans la partie rigide 36 de l'organe 14. Les moyens 44 ont la forme d'un cône partant du canal 42 et s'élargissant vers l'extrémité distale du dispositif, de façon à former une goutte et à éviter que le liquide soit distribué par jet, le cône 44 débouchant sur une portion cylindrique 46, permettant de calibrer la goutte.

L'élément de rappel 15 est, dans cet exemple, un ressort métallique en spirale. Cet élément 15 exerce une force de rappel sur l'organe d'étanchéité 14, en s'appuyant sur la surface 38 de la partie rigide 36, de façon à rappeler l'organe d'étanchéité 14 dans sa position de blocage du liquide.

Comme on peut le voir sur les figures, l'organe d'étanchéité 14 est fixé entre les deux parties 16, 18, de façon étanche, afin d'éviter que du liquide, passant par le canal 22, ne s'échappe à l'intérieur de l'enveloppe 18.

Le fonctionnement du dispositif de distribution de la figure 2 va à présent être décrit.

Lorsque l'utilisateur souhaite utiliser le dispositif, il ôte tout d'abord le capuchon du dispositif. Pour distribuer des gouttes de liquide, l'utilisateur actionne le dispositif, ce qui a pour effet d'augmenter la pression au sein du résevrvoir et de faire passer du liquide dans le canal 22, et donc d'exercer une pression sur la partie élastomère 34. Sous cette pression, l'organe d'étanchéité passe de sa position de blocage de liquide à sa position de libération de liquide, en effectuant une translation vers le haut, comme illustré par la flèche 48. Plus précisément, la zone 40 de la partie élastomère 34 se déforme, en s'allongeant, pour autoriser ce déplacement vers le haut de la partie élastomère. A l'issue de ce déplacement, l'étanchéité assurée par la coopération des moyens d'appui 20 avec l'organe d'étanchéité 14 est rompue, et le liquide peut passer à travers le canal 42 jusque dans les parties 44, 46, de façon à former une goutte de liquide. Le trajet du liquide est illustré par la flèche 50. Une fois la goutte libérée, l'utilisateur peut arrêter d'exercer la pression sur le réservoir, lequel se remplit d'air grâce au passage au canal 24. Par ailleurs, la pression du liquide sortant s'arrêtant, l'organe d'étanchéité 14 reprend sa position de blocage du liquide, sous l'effet de la force de rappel de l'élément 15. Ainsi, les moyens d'appui 20 et la partie élastomère de l'organe 14 coopèrent à nouveau de façon à bloquer la sortie de liquide. On notera que, dans cette position de blocage, l'organe 14 assure le blocage du liquide par compression de la partie 34 contre la partie rigide 36, cette compression étant assurée par les moyens 20.

On notera que l'exemple décrit peut présenter des variantes. En particulier, l'élément de rappel 15 est un ressort en spirale, mais on pourrait prévoir d'autres types d'éléments de rappel, en matière métallique ou pas, tels qu'une lame élastique ou un élément élastomère. Notamment, cet élément de rappel 15 pourrait être directement intégré dans l'organe d'étanchéité 14, en étant intégré soit dans la partie élastomère 34 soit dans la partie rigide 36, ou encore être intégré dans l'enveloppe 18.

Parmi les avantages du dispositif de la figure 2, on comprend que, comme les moyens de dosage 44, 46 sont ménagés sur l'organe 14, ils se trouvent au plus près de la zone d'étanchéité, délimitée par l'appui des moyens 20. Par ailleurs, comme ces moyens sont directement disposés sur l'organe 14, les risques de contamination par infiltration de liquide dans des parties "sales" (disposées en aval de la zone d'étanchéité) sont bien moins importants que dans le cas où ils seraient disposés sur une pièce non solidaire de l'organe 14, par exemple sur l'enveloppe 18. On notera que, sur cet exemple de la figure 2, les moyens de dosage sont ménagés sur la partie rigide 36, ce qui peut être plus facile à réaliser et assurer un dosage plus homogène que s'ils étaient faits sur la partie élastomère 34. Néanmoins, on pourrait également envisager des les ménager sur cette partie 34.

Parmi les autres avantages du dispositif de distribution de cet exemple, on notera en particulier que la partie rigide 36 constitue une sorte de coquille pour la partie élastomère 34, ce qui permet d'exercer plus facilement une contrainte sur l'organe d'étanchéité 14, sans risquer de le déformer.

Sur la variante de la figure 3, l'organe d'étanchéité est un élément pouvant être réalisé en élastomère ou en matériau plastique suffisamment souple, intégralement réalisé dans un même matériau, et l'embout ne présente pas de ressort. En effet dans cet exemple, l'organe 14 assure le blocage de liquide en étant monté déformé sur le dispositif. En effet l'organe 14 est en forme de chapeau, muni d'une forme cylindrique 65 et d'un rebord 66. La périphérie 68 de ce rebord est fixée de façon permanente entre les parties 16 et 18, de façon à assurer une étanchéité statique. Cette fixation est faite en déformant l'organe 14 : celui-ci est chaussé sur la protubérance 19 et déformé de façon que l'organe 14 est contraint de se plaquer contre la protubérance 19, plus précisément contre les moyens 20. En effet, la forme de l'organe 14 illustré sur la figure 3 est différente de la forme de l'organe 14 avant assemblage. Ce plaquage de l'organe 14 assure l'étanchéité dans la position de blocage. Ainsi, dans cet exemple de la figure 3, l'organe 14 assure le blocage du liquide par la déformation par flexion de l'organe 14, assurée par les moyens d'appui 20 disposées au droit d'une zone évidée.

Dans cet exemple de la figure 3, les moyens de dosage 44, 46 sont ménagés intégralement dans l'élément élastomère 14. Ces moyens 44, 46 sont analogues à ceux de la figure 2.

Le fonctionnement du dispositif de la figure 3 est analogue à celui de la figure 2.

## Revendications

1. Dispositif de distribution de doses prédéterminées de liquide, comportant un organe d'étanchéité (14) pouvant prendre une position de libération de liquide, laissant sortir du liquide hors du dispositif, et une position anti-retour empêchant le retour de liquide à l'intérieur du dispositif, dans lequel l'organe d'étanchéité (14) comporte des moyens de dosage du liquide à distribuer (44, 46), les moyens de dosage (44, 46) étant des moyens de formation de gouttes de liquide, le dispositif comportant un canal (42) de passage de liquide débouchant sur les moyens (44, 46) de formation de gouttes, **caractérisé en ce que** les moyens de formation de gouttes comprennent une forme évasée (44) depuis ce canal (42), le dispositif comprenant un embout (10) destiné à être rapporté sur un réservoir en matière plastique contenant le liquide à distribuer, l'embout (10) comprenant l'organe d'étanchéité (14), une première partie (16) et une deuxième partie (18), l'organe d'étanchéité (14) étant disposé entre la première partie (16) et la deuxième partie (18).

2. Dispositif selon la revendication 1, comportant des moyens (20) d'appui sur l'organe d'étanchéité, au droit d'une zone évidée, assurant la déformation par flexion de l'organe d'étanchéité (14) pour qu'il prenne sa position anti-retour.

3. Dispositif selon la revendication 1 ou 2, dans lequel l'organe d'étanchéité est au moins partiellement souple, et le dispositif comporte des moyens (20) d'appui sur l'organe d'étanchéité, assurant la déformation par compression de la partie souple (34) de l'organe d'étanchéité pour qu'il prenne sa position anti-retour.

4. Dispositif selon l'une quelconque des revendications précédentes, dans lequel la forme évasée (44) débouche sur une forme sensiblement cylindrique (46).

5. Dispositif selon l'une quelconque des revendications 1 à 4, dans lequel l'organe d'étanchéité (14) est un élément élastomère, intégralement réalisé en matériau élastomère.

6. Dispositif selon l'une quelconque des revendications 1 à 4, dans lequel l'organe d'étanchéité (14) comprend une partie élastomère (34) et une partie rigide (36), ces parties étant solidaires l'une de l'autre en déplacement.

7. Dispositif selon la revendication précédente, dans lequel les moyens de dosage (44, 46) sont réalisés dans la partie rigide (36).

8. Dispositif selon l'une quelconque des revendications précédentes, dans lequel la partie de l'organe d'étanchéité comportant les moyens de dosage (44, 46) est disposée au voisinage immédiat d'un orifice (64) de libération du liquide hors du dispositif.

## Patentansprüche

1. Vorrichtung zur Ausgabe vorgegebener Dosen von Flüssigkeit, mit einem Dichtungselement (14), das eine Flüssigkeitsabgabeposition, die Flüssigkeit aus der Vorrichtung herauslässt, und eine Rückflusssperrposition, die den Rückfluss von Flüssigkeit ins Innere der Vorrichtung verhindert, einnehmen kann, wobei das Dichtungselement (14) Mittel zur Dosierung der auszugebenden Flüssigkeit (44, 46) aufweist, wobei die Dosierungsmittel (44, 46) Mittel zur Bildung von Flüssigkeitstropfen sind, wobei die Vorrichtung einen Flüssigkeitsdurchflusskanal (42) aufweist, der in die Tropfenbildungsmittel (44, 46) mündet, **dadurch gekennzeichnet, dass** die Tropfenbildungsmittel eine sich von diesem Kanal (22) aus aufweitende Form (44) umfassen, wobei die Vorrichtung eine Düse (10) aufweist, die dazu bestimmt ist, an einem Kunststoffbehälter befestigt zu werden, der die auszugebende Flüssigkeit enthält, wobei die Düse (10) das Dichtungselement (14), einen ersten Teil (16) und einen zweiten Teil (18) aufweist, wobei das Dichtungselement (14) zwischen dem ersten Teil (16) und dem zweiten Teil (18) angeordnet ist.

2. Vorrichtung nach Anspruch 1, mit Mitteln (20) zur Anlage am Dichtungselement, in Entsprechung zu einem ausgehöhlten Bereich, die die Biegeverformung des Dichtungselements (14) gewährleisten, damit es seine Rückflusssperrposition einnimmt.

3. Vorrichtung nach Anspruch 1 oder 2, wobei das Dichtungselement zumindest teilweise flexibel ist, und die Vorrichtung Mittel (20) zur Anlage am Dichtungselement aufweist, die die Kompressionsverformung des flexiblen Abschnitts (34) des Dichtungselements gewährleisten, damit es seine Rückflusssperrposition einnimmt.

4. Vorrichtung nach einem der vorstehenden Ansprüche, wobei die sich aufweitende Form (44) in eine im Wesentlichen zylindrische Form (46) mündet.

5. Vorrichtung nach einem der Ansprüche 1 bis 4, wobei das Dichtungselement (14) ein elastomeres Element ist, das vollständig aus elastomerem Material hergestellt ist.

6. Vorrichtung nach einem der Ansprüche 1 bis 4, wobei das Dichtungselement (14) einen elastomeren Abschnitt (34) und einen steifen Abschnitt (36) umfasst, wobei diese Abschnitte verlagerungsfest miteinander verbunden sind.

7. Vorrichtung nach dem vorstehenden Anspruch, wobei die Dosierungsmittel (44, 46) im steifen Abschnitt (36) ausgebildet sind.

8. Vorrichtung nach einem der vorstehenden Ansprüche, wobei der die Dosierungsmittel (44, 46) aufweisende Abschnitt des Dichtungselements in unmittelbarer Nähe einer Öffnung (64) zur Abgabe der Flüssigkeit aus der Vorrichtung angeordnet ist.

## Claims

1. A device for dispensing predetermined quantities of liquid, which device includes a sealing member (14) that can take up a liquid release position, allowing liquid to flow out of the device, and a non-return position preventing liquid from flowing back into the device, wherein the sealing member (14) is provided with metering means (44, 46) for the liquid to be dispensed, the metering means (44, 46) being liquid drop-forming means, the device including a liquid-passing channel (42) opening out into the drop-forming means (44, 46), **characterised in that** the drop-forming means comprise a flared shape (44) that flares from said channel (42), which device comprises an end piece (10) intended to be connected to a plastic tank containing the liquid to be dispensed, the end piece (10) comprising the sealing member (14), a first portion (16) and a second portion (18), the sealing member (14) being disposed between the first portion (16) and the second portion (18).

2. A device according to claim 1, including bearing means (20) for bearing against the sealing member, in line with a recessed zone, serving to deform the sealing member (14) by bending deformation so that it takes up its non-return position.

3. A device according to claim 1 or claim 2, wherein the sealing member is flexible at least in part, and the device includes bearing means (20) for bearing against the sealing member, serving to deform the flexible portion (34) of the sealing member by compression deformation so that it takes up its non-return position.

4. A device according to any preceding claim, wherein the flared shape (44) opens out into a substantially cylindrical shape (46).

5. A device according to any one of claims 1 to 4, wherein the sealing member (14) is an elastomer element that is made entirely of elastomer.

6. A device according to any one of claims 1 to 4, wherein the sealing member (14) comprises an elastomer portion (34) and a rigid portion (36), said portions being constrained to move with each other.

7. A device according to the preceding claim, wherein the metering means (44, 46) are formed in the rigid portion (36).

8. A device according to any preceding claim, wherein the portion of the sealing member that is provided with the metering means (44, 46) is disposed in the immediate vicinity of an orifice (64) for releasing liquid from the device.
